# EUROPEAN PATENT APPLICATION

(11) **EP 3 287 467 A1**
(43) Date of publication of application: **28.02.2018**
(21) Application number: 16185193.6
(22) Date of filing: 22.08.2016
(51) Int. Cl.: C07J 9/00, C07J 51/00

(54) **PREPARATION OF OBETICHOLIC ACID COMPRISING CONTINUOUS FLOW PROCESS STEPS**

(71) Applicant: ratiopharm GmbH, 89079 Ulm (DE)
(72) Inventor: Coutable, Ludovic, 73230 Kirchheim unter Teck (DE)
(74) Representative: Ter Meer Steinmeister & Partner

(57) **Abstract**

The present invention relates to a process for the preparation of obeticholic acid or one of its process intermediates, the process comprising one or more continuous flow process steps. Further, the present invention relates to the use of a continuous flow reactor for the preparation of obeticholic acid or one of its process intermediates. In particular, the present invention relates to a process for the preparation of obeticholic acid from 3α-hydroxy-7-keto-5β-cholanic acid (7-KCA) as starting material comprising one or more continuous flow process steps.

## Description

The present invention relates to a process for the preparation of obeticholic acid or one of its process intermediates, the process comprising one or more continuous flow process steps. Further, the present invention relates to the use of a continuous flow reactor for the preparation of obeticholic acid or one of its process intermediates. In particular, the present invention relates to a process for the preparation of obeticholic acid from 3α-hydroxy-7-keto-5β-cholanic acid (7-KCA) as starting material comprising one or more continuous flow process steps.

### Background of the invention

Obeticholic acid (OCA, lab-code: INT-747) is a bile acid derivative and potent and selective agonist of the farnesoid X receptor (FXR). FXR controls bile acid homeostasis and its activation leads to an inhibition of the conversion of cholesterol into bile acid and prevents from the accumulation in the liver. Obeticholic acid is currently evaluated for the treatment of alcoholic hepatitis, of nonalcoholic steatohepatitis (NASH) and of primary biliary cirrhosis.

In the context of this invention, the term "obeticholic acid" is understood to mean 6α-ethyl-chenodeoxycholic acid (also: 3α,7α-dihydroxy-6α-ethyl-5β-cholan-24-oic acid) in accordance with the following chemical formula (1).

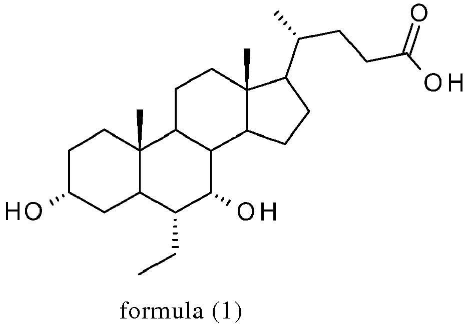

The compound can be present in the form of different stereoisomers. In the context of this invention, obeticholic acid is preferably present in the form of (4R)-4-[(3R,5S,6R,7R,8S,9S,10S,13R,14S,17R)-6-ethyl-3,7-dihydroxy-10,13-dimethyl-2,3,4,5,6,7,8,9,11,12,14,15,16,17-tetradecahydro-1H-cyclopenta[a]phenanthren-17-yl]pentanoic acid, as depicted in the above formula (1).

Synthesis pathways for amorphous obeticholic acid, a crystalline obeticholic acid Form C and the therapeutic use of obeticholic acid have been described in WO 2013/192097. An alternative synthesis pathway for the preparation of obeticholic acid is described in CN 105315320 A.

However, the processes known in the art are performed as batch processes, having the disadvantages of inefficient synthesis steps, such as performing the reactions at limited reaction temperatures, which leads to long durations of reaction, incomplete reactions and low product yield. Further, performing the synthesis of obeticholic acid in batch reactors has the disadvantage of needing separate reactors for each of the process steps. Therefore, batch processes have high requirements of reaction setup while the reaction efficiency is limited.

### Technical problem

Therefore, there is a need to overcome the drawbacks of the prior art batch processes. In particular, there is a need in the art for improved processes for preparing obeticholic acid.

It is thus an object of the present invention to provide a process for preparing obeticholic acid with an easier reaction setup while at the same time increasing reaction efficiency. In particular, the processes for preparing one of the key intermediates of obeticholic acid of 3a-hydroxy-7-oxo-5β-cholan-24-oic acid C₁₋₄alkyl ester (1), 3α,7α-ditrimethylsilyloxy-5β-chol-6-en-24-oic acid C₁₋₄alkyl ester (2), 3a-hydroxy-6-ethyliden-7-oxo-5β-cholan-24-oic acid C₁₋₄alkyl ester (3), and/or 3α-hydroxy-6-ethyliden-7-oxo-5β-cholan-24-oic acid (4) should be improved.

### Summary of the invention

It has surprisingly been found in the present invention that the prior art technical problems could be solved by performing one or more process steps in the process for preparing obeticholic acid as continuous flow process steps.

In a first aspect, the present invention provides a process for the preparation of 3α,7β-dihydroxy-6a-ethyl-5β-cholan-24-oic acid (obeticholic acid) or one of its process intermediates, the process starting from 3α-hydroxy-7-keto-5β-cholanic acid (7-KCA) as starting material and comprising one or more continuous flow process steps.

In another aspect, the present invention is directed to use of a continuous flow reactor for the preparation of obeticholic acid or one of its process intermediates.

### Brief description of the Figures

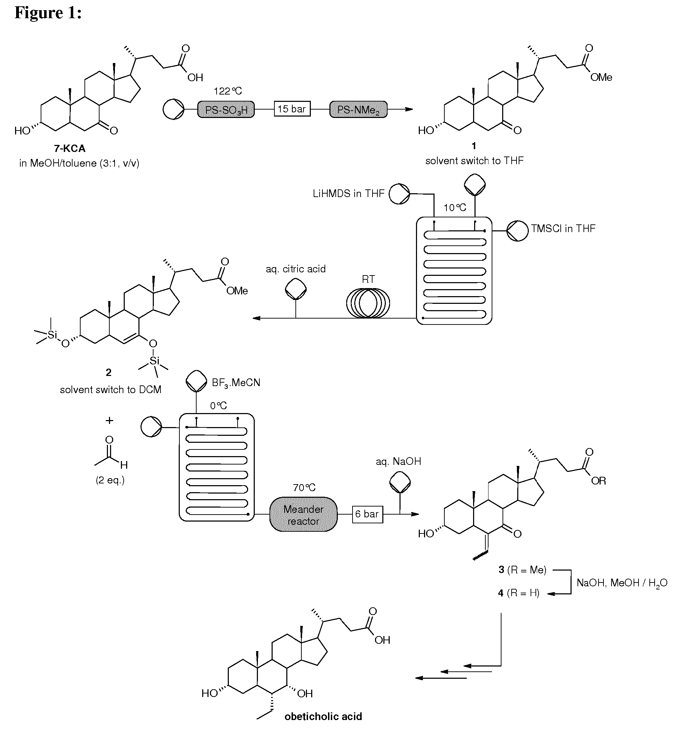

Figure 1: Schematic representation of an exemplary process for preparing obeticholic acid according to the present invention, the process comprising continuous flow steps.
Figure 2: Experimental setup for the esterification of 7-KCA with methanol, process step (I).
Figure 3: Optimized experimental setup for the esterification of 7-KCA with methanol, process step (I).
Figure 4: Experimental setup for the formation of compound (2) under continuous flow, process step (II).
Figure 5: Experimental setup for the synthesis of compound (3) under continuous flow, process step (III).
Figure 6: Synthesis of compound (4) via saponification of methyl ester (3), process step (IV).
Figure 7: Synthesis of 3α-hydroxy-6α-ethyl-7-keto-5β-cholanic acid from compound (4), process step (V).

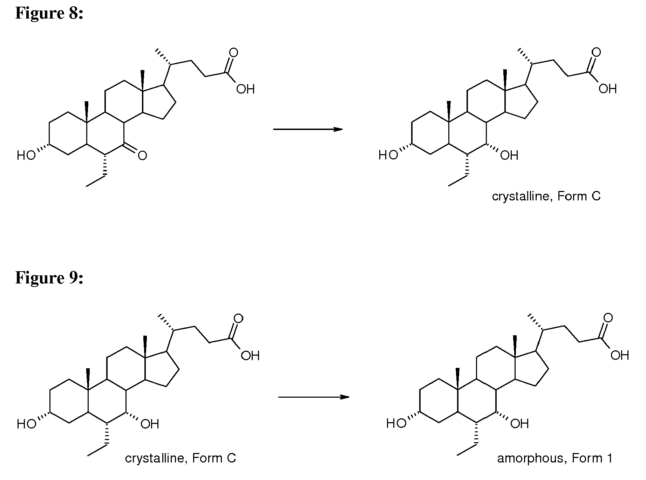

Figure 8: Synthesis of crystalline obeticholic acid, Form C, process step (VI).
Figure 9: Synthesis of amorphous obeticholic acid, Form 1, process step (VII).

### Detailed description of the invention

The present invention relates to a process for the preparation of obeticholic acid or one of its process intermediates selected from 3a-hydroxy-7-oxo-5β-cholan-24-oic acid C₁₋₄alkyl ester (1), 3α,7α-ditrimethylsilyloxy-5β-chol-6-en-24-oic acid C₁₋₄alkyl ester (2), 3a-hydroxy-6-ethyliden-7-oxo-5β-cholan-24-oic acid C₁₋₄alkyl ester (3) and 3α-hydroxy-6-ethyliden-7-oxo-5β-cholan-24-oic acid (4), the process comprising one or more continuous flow process steps. In a particular embodiment, the process starts from 3α-hydroxy-7-keto-5β-cholanic acid (7-KCA) as starting material.

Typically, the process according to the present invention starts from 3α-hydroxy-7-keto-5β-cholanic acid (7-KCA) and involves preparation of the one or more of the key intermediates of obeticholic acid as represented by formulae (1) to (4) below. Therefore, the process of the present invention may comprise one or more of the following process steps provided that at least one of the steps is performed as a continuous flow process step:
(I) Esterification of 3α-hydroxy-7-keto-5β-cholanic acid (7-KCA) with a C₁₋₄alkyl alcohol to form 3a-hydroxy-7-oxo-5β-cholan-24-oic acid C₁₋₄alkyl ester (1),
(II) Reacting (1) with a silylation agent and a lithium amide base to form 3α,7α-ditrimethylsilyloxy-5β-chol-6-en-24-oic acid C₁₋₄alkyl ester (2),
(III) Reacting (2) with acetaldehyde or acetaldehyde acetal and a Lewis acid catalyst to form 3a-hydroxy-6-ethyliden-7-oxo-5β-cholan-24-oic acid C₁₋₄alkyl ester (3),
(IV) Reacting (3) with NaOH to form 3α-hydroxy-6-ethyliden-7-oxo-5β-cholan-24-oic acid (4), and
(V) Converting (4) into obeticholic acid.

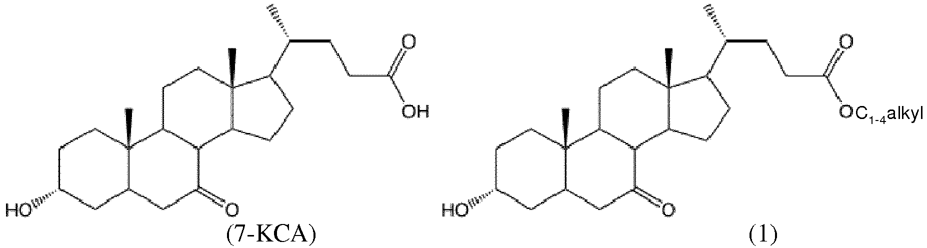

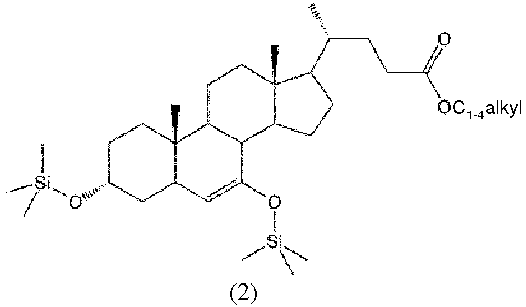

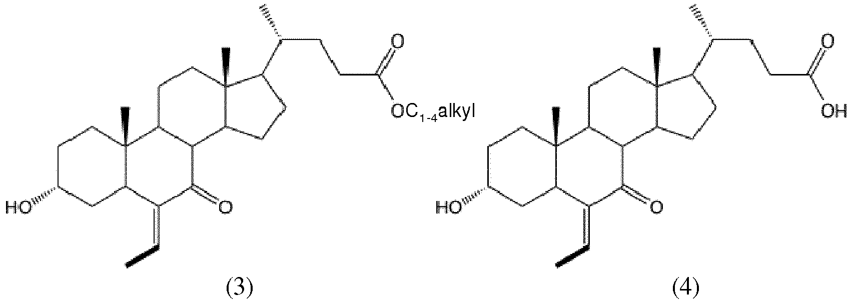

Preferably, the C₁₋₄alkyl alcohol is methanol to form 3α-hydroxy-7-oxo-5β-cholan-24-oic acid methyl ester, but may alternatively be selected from ethanol, n-propanol, isopropanol or n-butanol or isobutanol.

As mentioned above, one or more of steps (I), (II), (III) and (IV) may be performed in the present invention as continuous flow process steps. Preferably, one or more of steps (I), (II) and (III) are performed as continuous flow process steps. In a particularly preferred embodiment of the present invention, at least two of steps (I), (II) and (III) are performed as continuous flow process steps. Most preferably, steps (I) to (III) are performed as continuous flow process steps.

### Reactor for performing the continuous flow process steps

The continuous flow process steps as performed in the process of the present invention may be performed in any reactor suitable for performing continuous flow reactions. Preferably, the processes of the present invention are performed in micro-reactors such as those designed for small-, i.e. micro- or nano-scale synthesis of chemical products. Such micro-reactors are for example available from Ehrfeld Mikrotechnik BTS, the Fraunhofer Initiative FAMOS*flexible*, Cellular Process Chemistry CPC GmbH, or Lonza AG, which all market micro-reactor modular systems. A preferred micro-reactor system is the Modular MicroReaction System by Ehrfeld Mikrotechnik BTS, which allows performing temperature-controlled chemical reactions and thus is especially suitable for the synthesis reactions in the preparation of obeticholic acid.

Such modular micro-reactor systems are for example described in WO 2007/112945 A1, WO 00/62919 A1 and WO 2006/010490 A1, which are all hereby incorporated by reference in their entirety.

In continuous reaction technology, typically a plurality of feeds or reactants continuously flowing into a reactor or micro-reactor chemically interact therein to form a product that continuously flows out of it. The term "*flow device*" as used in the present invention thus encompasses the one or more reactors for conducting the processes of the present invention, as well as the fluid channel system comprising the one or more inlets and outlets to allow a continuous flow of substances, as well as any other means, such as pumps, heating means, or pressure means, and the like, which may be necessary to conduct the processes as described herein.

Within the reactor, i.e. the continuous reactor or continuous flow reactor, there can be provided a process fluid channel system bringing together, mixing and swirling the plurality of feeds embedded in an optimum reaction environment, especially a characteristic temperature regime, for the chemical reactions to take place. The process fluid channel system may be divided into at least one turbulent-flow mixing zone and at least one essentially laminar-flow retention zone that are appropriately arranged in series. In case of more than one mixing zone and/or retention zone, they are concatenated in a suitable manner. In order to establish a well determined temperature regime, a heat exchange system, for example in the form of channels, is generally integrated.

A reactor or micro-reactor of the above described type is disclosed, for example, in US 2012/076705 A1 and EP 1 839 739 A1, which are all hereby incorporated by reference in their entirety, and which relate to a modular micro-reactor comprising a plurality of process modules and heat exchange modules. The process modules may be connected externally to produce a flow-channel system by adding the individual subsystems, and due to heat exchange modules, a section-wise heating or cooling of the chemical substances (reactant(s), product(s)) flowing in the flow-channel system can be achieved.

Typically, a reactor or micro-reactor for conducting the process of the present invention is of modular structure. The reactor typically comprises one or more reaction units comprising a process fluid channel system for continuous reaction of a plurality of feeds or reactants flowing into the reaction unit to form at least one product flowing out of the reaction unit, and a heat exchange fluid channel system for adjusting the temperature environment of the process fluid channel system.

Further, a process fluid channel system of a typical reactor for use in the present invention may have a plurality of primary inlet ports for a plurality of primary feeds flowing into the process module, and at least one secondary inlet port provided after said plurality of primary inlet ports in a flow direction of the chemical substances for at least one secondary feed stream flowing into the process module. Consequently, complex chemical reactions can be observed to take place along the process fluid channel system. For example, a first reaction may be initiated by mingling two primary substances to form a first (intermediate) product, and then a secondary feed stream can be added and mixed with the first (intermediate) product to form a second intermediate product etc. Each time, a secondary feed is added, it is advantageously mixed with the respective product previously formed.

A conventional reactor or micro-reactor may further comprise one or more mixing modules for receiving and mixing at least two reactive fluids; and optionally at least one thermal adjusting module typically disposed upstream of said mixing module for adjusting a temperature of said reactive fluids prior to entering said mixing module; and at least one retention module disposed downstream of the mixing module for accommodating the reactive fluid mixture. Using more than one mixing module allows to sequentially introducing more reactive fluids for sequential reaction steps.

The temperature of the reactive fluids in the mixing module(s) can be controlled by one or more heat exchange modules adjacent to said mixing module(s). A warm or cold heat exchange fluid is supplied to the at least one heat exchange fluid passage within each of the heat exchange module(s), which supplies or removes heat from the process module by heat transfer.

Therefore, before mixing of two or more reactive fluids, said reactive fluids may be heated or cooled by such heat-controlling means. One or more thermal adjusting modules may be provided upstream of said mixing module. Said thermal adjusting/controlling module comprises at least one reactive fluid passage for each reactive fluid to be heated or cooled. While flowing through said reactive fluid passage(s) each reactive fluid is heated or cooled by the heat exchange modules adjacent to said thermal adjusting module. By providing different passage volumes it becomes possible to heat or cool the different reactants differently.

The reactors used for conducting the continuous flow process steps are typically equipped with one or more pumping devices, such as hydraulic pumps, syringe pumps, HPLC pumps, or the like, for allowing the substances used and/or produced in the processes of the present invention to be fed into, through and out of the reactors, thus enabling fluid passages and a continuous streaming of the substances. Therefore, in the context of the present invention, the term "feed(ing)" and/ or "supply(ing)" preferably means "pump(ing)".

The reactors used for conducting the continuous flow process steps are further typically equipped with one or more pressure regulators for allowing to conduct the processes of the present invention under pressure (back-pressure regulator).

In a further aspect, the present invention relates to the use of a continuous flow reactor or micro-reactor as described above for the preparation of 3α,7β-dihydroxy-6α-ethyl-5β-cholan-24-oic acid (obeticholic acid) or one of its process intermediates selected from 3a-hydroxy-7-oxo-5β-cholan-24-oic acid C₁₋₄alkyl ester (1), 3α,7α-ditrimethyl-silyloxy-5β-chol-6-en-24-oic acid C₁₋₄alkyl ester (2), 3α-hydroxy-6-ethyliden-7-oxo-5β-cholan-24-oic acid C₁₋₄alkyl ester (3) and 3α-hydroxy-6-ethyliden-7-oxo-5β-cholan-24-oic acid (4).

### Esterification of 3α-hydroxy-7-keto-5β-cholanic acid (7-KCA) to form 3α-hydroxy-7-oxo-5β-cholan-24-oic acid C1-4alkyl ester (1)

Step (I) of the invention comprises esterification of 7-KCA to form 3α-hydroxy-7-oxo-5β-cholan-24-oic acid C₁₋₄alkyl ester (1) by reacting 7-KCA with an alcohol having 1-4 carbon atoms in the presence of a heterogeneous catalyst.

Esterification is typically conducted in a flow device comprising at least one feed solution inlet, a heatable cartridge containing a heterogeneous catalyst and a backpressure regulator. In this process step, 3α-hydroxy-7-keto-5β-cholanic acid (7-KCA) in a mixture of alcohol and organic solvent are supplied to the flow reactor inlet.

The heterogeneous catalyst is typically a solid-support catalyst, preferably, a cation exchange resin containing sulfonic acid functional groups. Such heterogeneous catalysts are commercially available and include, for example, DOWEX^{®} 50WX8 or Amberlyst^{®} 15.

The alcohol used for esterification may be selected from one or more of methanol, ethanol, *n*-propanol or *n*-butanol, preferably methanol. As solubility of 7-KCA in methanol is about 30 g/L (0.076 M) at room temperature (25°C), higher concentrations of 7-KCA can be reached by adding an organic solvent, such as an ether or aromatic hydrocarbon, i.e. by adding a co-solvent which is used in addition to the alcohol. Preferred organic solvents are tetrahydrofuran (THF), 2-methyl tetrahydrofuran (2-MeTHF), anisole, xylene and toluene, which may be used alone or in admixture of two or more of them, preferably toluene.

In a preferred embodiment, the alcohol is methanol and the organic solvent is toluene. The ratio of the alcohol and the organic solvent, such as the ratio of methanol:toluene is from 1:1 to 10:1, more preferably from 2:1 to 6:1 and most preferably is from 2.5:1 to 4:1.

A typical flowrate for performing the reaction in a micro-reactor system as described above is from 0.2 to 4 ml/min, preferably from 0.5 to 2 ml/min, more preferably from 0.6 to 1 ml/min.

Typically, the reaction mixture leaving the reactor of step (I) is supplied to, preferably circulated through, a cartridge filled with a basic anion exchanger containing tertiary C₁₋₁₀-alkyl amine functional groups, preferably C₁₋₄-alkyl amine functional groups, such as methyl amine, ethyl amine or propyl amine functional groups, most preferably methyl amine functional groups. The use of such basic anion exchangers has the purpose of removing residual amounts of unreacted carboxylic acid 7-KCA from the process stream, and thus they act as scavengers during the reaction. Preferred scavengers for this purpose are weak base anion exchangers, for example anion exchangers containing alkyl amine groups. Such weak base anion exchangers are commercially available and include, for example, Amberlite® IRA-96, Amberlyst® A-21, or DOWEX® M 43.

The process of step (I) is typically performed in a temperature-controlled reactor, which allows the reaction to be performed at a temperature of between 70°C and 200°C, preferably a temperature of from 100°C to 140°C. Further preferably, the reaction of the process of step (I) is performed under elevated pressure, such as a pressure of more than 2 bar and up to 20 bar. Preferably, the reaction is performed under a pressure of from 10 bar to 15 bar.

Therefore, the present invention allows performing the esterification of 3α-hydroxy-7-keto-5β-cholanic acid (7-KCA), i.e. process step (I), at much higher temperatures than described in the prior art WO 2013/192097, wherein esterification is carried out in a batch reactor for three hours at a temperature of 62 to 64°C. Hence, the present invention allows improving reaction efficiency by increasing the reaction temperature to thereby considerably shorten the reaction time and improve product yield.

### Silylation of (1) to form 3α,7α-ditrimethylsilyloxy-5β-chol-6-en-24-oic acid C₁₋₄alkyl ester (2)

Silylation in process step (II) comprises reacting 3a-hydroxy-7-oxo-5β-cholan-24-oic acid C₁₋₄alkyl ester (1) with a silylation agent and a lithium amide base. Typically, the silylation reaction is conducted in a flow device comprising at least one temperature-controlled reactor unit with at least three feed solution inlets, a second residence reactor unit and a 3-way connection module to perform inline quenching of the reaction.

In a typical continuous reactor arrangement, 3a-hydroxy-7-oxo-5β-cholan-24-oic acid C₁₋₄alkyl ester (1) in an organic solvent is supplied to a first flow reactor inlet; the silylation agent in an organic solvent is supplied to a second flow reactor inlet; lithium amide base in an organic solvent is supplied to a third flow reactor inlet; and an acidic solution is supplied to a 3-way connection module.

The silylation agent in step (II) is preferably chlorotrimethylsilane.

The lithium amide base may be selected from one or more of lithium hexamethyldisilazide, lithium diisopropylamide and lithium 2,2,6,6-tetramethylpiperidide. Preferably, a combination of chlorotrimethylsilane and lithium hexamethyldisilazide is used for the silylation reaction.

The organic solvent may be selected from one or more of tetrahydrofuran, 2-methyl tetrahydrofuran, toluene, xylene or anisole.

The acidic solution is preferably a citric acid solution, typically an aqueous solution, but may alternatively be any aqueous solution of weak acidity, such as an acidic solution of an acid having a pKa of 2 or higher, such as acetic acid solution, phosphoric acid solution or tartaric acid solution.

Typically, the process of step (II) is performed at a temperature of from -20°C to 40°C, preferably of from 10°C to 25°C.

Advantages of the continuous process of process step (II) of the present invention over the prior art batch process described in WO 2013/192097 are a much easier reaction setup (e.g. avoid the use of two separate reactors) and that the reaction can be performed at much higher temperatures instead of -20 to -25°C used in WO 2013/192097. Hence, the present invention allows improving reaction efficiency by increasing the reaction temperature to thereby considerably shorten the reaction time and improve product yield.

### Reaction of (2) to form 3α-hydroxy-6-ethyliden-7-oxo-5β-cholan-24-oic acid C₁₋₄alkyl ester (3)

Reaction step (III) is a process for the continuous flow Mukaiyama aldol reaction of compound (2) with acetaldehyde or acetaldehyde acetal in the presence of a Lewis acid catalyst.

The reaction is typically conducted in a flow device comprising at least one preferably temperature-controlled reactor unit with at least two feed solution inlets, a second preferably temperature-controlled residence reactor unit and a 3-way connection module to perform inline quenching of the reaction. A residence reactor as used in the process of step (III) is also commonly known as Meander reactor.

The reaction may for example be carried out in a micro-reaction system such as the Modular MicroReaction System from Ehrfeld Mikrotechnik BTS. The system may consist of a continuous flow reactor such as the FlowPlate^{®} Lab reactor associated to a residence reactor, both temperature-controlled by means of external thermostats. The system can be pressurized by use of a back-pressure regulator. Reactants solution and the solution of the Lewis acid catalyst, such as the boron trifluoride, can be circulated with syringe pumps; the quenching solution may be introduced in the system via an HPLC pump.

Typically, 3α,7α-ditrimethylsilyloxy-5β-chol-6-en-24-oic acid C₁₋₄alkyl ester (2) and acetaldehyde or acetaldehyde acetal in an organic solvent are pumped to a first flow reactor inlet; the Lewis acid catalyst in an organic solvent is pumped to a second flow reactor inlet; and sodium hydroxide aqueous solution is pumped to the 3-way connection module.

NaOH or any other strong base such as KOH may be used in step (III) for the destruction of BF₃ as present during the reaction as catalyst.

The acetaldehyde acetal in step (III) may be selected from acetaldehyde dimethyl acetal or acetaldehyde diethyl acetal. Further, the Lewis acid catalyst may be selected from one or more of boron trifluoride complex with an organic solvent or titanium tetrachloride. The organic solvent may be selected from one or more of dichloromethane, acetonitrile, tetrahydrofuran, 2-methyl tetrahydrofuran, toluene, xylene or anisole, and preferably is dichloromethane.

As mentioned above, the reaction in step (III) is preferably performed in temperature-controlled reactors. The reaction temperature of step (III) in the first reactor unit is typically from -40°C to 60°C, preferably from -20°C to 25°C. The reaction temperature in the second reactor unit, i.e. the residence reactor, is typically from 25°C to 120°C, preferably from 50°C to 90°C.

A typical flowrate for performing the reaction as described above is from 0.2 to 4 ml/min, preferably from 0.3 to 2 ml/min, more preferably from 0.4 to 1 ml/min.

The pressure is set in the flow device typically by using a backpressure regulator, which may be placed between the reactor unit(s) and the 3-way connection module, to maintain the solvent under its boiling point if the reaction temperature of step (III) in the second reactor unit is above the boiling point of the solvent at 1013 hPa. Typically, the reaction is conducted under a pressure of from 2 bar to 10 bar, preferably from 4 bar to 8 bar, depending on the boiling point of the solvent used in the reaction.

Advantages of performing process step (III) as continuous process over the batch process from WO 2013/192097 are a much easier reaction setup (avoid the use of two separate reactors) and that the reaction can be performed at increased and convenient temperatures compared to the low-temperature reaction at -60 to -65°C of WO 2013/192097.

### Reacting (3) to form 3α-hydroxy-6-ethyliden-7-oxo-5β-cholan-24-oic acid (4)

Process step (IV) typically comprises reacting (3) with an inorganic base, such as NaOH, in the presence of a mixture of water and methanol, followed by acidification with citric acid. Other bases than NaOH, such as KOH, LiOH or Ba(OH)₂, and other acids than citric acid having a pKa of 2 or below, such as hydrochloric acid or phosphoric acid could also be used.

### Conversion of (4) to obeticholic acid (OCA)

The further process steps starting from 3a-hydroxy-6-ethyliden-7-oxo-5β-cholan-24-oic acid (4) and leading to the formation of obeticholic acid may be performed as described in the art, such as in WO 2013/192097. Further, one or more of the process steps may be carried out as continuous process steps, as described above.

These further process steps may include the reaction of 3α-hydroxy-6-ethyliden-7-oxo-5β-cholan-24-oic acid (4) with Pd/C and hydrogen gas to form 3α-hydroxy-6α-ethyl-7-keto-5-cholan-24-oic acid. The reaction can be carried out in one phase (hydrogenation and isomerization together) or in two phases (hydrogenation followed by isomerization). The reaction may be carried out at a temperature at 90 °C to 110 °C and at a pressure at 4 to 6 bars. During workup, the organic phase of the reaction mixture may be treated with activated carbon. The hydrogenation reaction mixture may be allowed to stir for 1 hour to 5 hours.

The next step may include the reaction of 3α-hydroxy-6α-ethyl-7-keto-5p-cholan-24-oic acid with NaBH₄ to form obeticholic acid. The reaction may be carried out at a temperature of 85°C to 110°C in a basic aqueous solution. Typically, the basic aqueous solution is an aqueous NaOH solution, which may be a mixture of 50% wt. NaOH solution and water. The reaction mixture is typically stirred for 3 hours to 5 hours.

For the workup, after the reaction is complete, the mixture may be cooled to about 80°C and transferred to a cooled reactor, which may have a temperature of 20°C to 60°C, and n-butyl acetate and an acid, such as citric acid, are added. The aqueous phase may be separated and discarded after checking the pH-value to ensure acidity. The organic phase containing the product may be concentrated, e.g. by distillation. The crystalline product may be dried, for example under vacuum at about 60 °C.

Obeticholic acid may be obtained by the above-described procedures in crystalline or amorphous form. Preferably, obeticholic acid is obtained in crystalline form, such as obeticholic acid Form C. Optionally, the crystalline obeticholic acid may subsequently be converted into amorphous obeticholic acid (Form 1).

### Examples

### Materials

Continuous flow systems were based on the Modular MicroReaction System from Ehrfeld Mikrotechnik BTS. Reagents/solvent(s) were delivered by either Knauer HPLC pumps (Smartline 100) or Sykam syringe pumps (S1610). Automation of the Modular MicroReaction System was done by the LabBox^{®} device, developed by Hitec Zang, consisting of a control unit and the associated visualisation and automation software package, called LabVision^{®}.

Standard reagents and solvents were purchased from TCI, Sigma-Aldrich and Merck. 3α-Hydroxy-7-keto-5β-cholanic acid (7-KCA) was purchased from Finetech Chemical.

### Analytical Methods

**¹H-NMR Spectroscopy**

| | |
|---|---|
| Instrument: | Varian Mercury 400 Plus NMR Spectrometer, Oxford AS, 400 MHz |

**IR-spectroscopy**

| | |
|---|---|
| Instrument: | Thermo Nicolet, Avatar 330 FT-IR. Smart Endurance Diamond-ATR |
| Software: | Omnic Vers. 6.1a |

**HPLC/UV-Method for In-Process Control and Intermediates Analyses**

| | | | |
|---|---|---|---|
| Method OCA01.M : | | | |
| Instrument: | Agilent 1200 | | |
| Column: | Phenomenex Kinetex C18, 150*4.6 mm; dp = 2.6 µm | | |
| Flow: | 1.5 ml/min | | |
| Temperature: | 40.0°C | | |
| Eluent | | | |
| A: | acetonitrile | | |
| B: | 0.1% H₃PO₄, pH 2.3 | | |
| Gradient: | Time [min] | eluent A [%] | eluent B [%] |
| | 0.0 | 55 | 45 |
| | 8.0 | 70 | 30 |
| | 9.1 | 55 | 45 |
| Stop time | 14.0 | | |
| Injection volume: | 10 µl | | |
| Detection: | DAD (λ = 200 and 210 nm) | | |

**LC-MS**

| | | | |
|---|---|---|---|
| HPLC: | | | |
| Instrument: | Agilent 1100 coupled with Esquire HCT (Brucker Daltonics) | | |
| Method: | OBETICHOLIC ACID-PH2-POLAR-100-5.M | | |
| Column: | Phenomenex Synergie Polar RP 100*3mm 2.5u | | |
| Flow: Temperature: Solvents: | 1.0 ml/min 40°C | | |
| Solvent A: | Acetonitrile | | |
| Solvent B: | formic acid pH 2.5 | | |
| Gradient: | Time [min] | eluent A [%] | eluent B [%] |
| | 0.0 | 40 | 60 |
| | 8.0 | 85 | 50 |
| | 9.0 | 95 | 5 |
| | 11.0 | 95 | 5 |
| | 11.1 | 40 | 60 |
| Stop time | 17.0 | | |
| Injection volume: | 3 µl | | |
| Detection: | DAD (λ = 218, 242 nm) | | |

**Mass Spectrometry:**

| | |
|---|---|
| Instrument: | Bruker HCT |
| Ion polarity: | Positive |
| Ion Source type: | ESI |
| Nebulizer gas: | 65 psi |
| Dry Gas: | 8 l/min. |
| Temperature: | 320°C |
| Scan [m/z] | 50 - 850 amu |

### Step I: Synthesis of 3α-Hydroxy-7-oxo-5β3-cholan-24-oic acid (1) via esterification of 7-KCA with methanol under continuous flow conditions

### Reactor cartridge preparation.

Amberlyst 15 was allowed to swell in methanol/toluene mixture (3:1, v/v). The wet resin was charged into the 5 mL stainless steel cartridge.

### Scavenger cartridge preparation.

Amberlyst^{®} A-21 was allowed to swell in methanol/toluene mixture (3:1, v/v). The wet resin was charged into a polypropylene *cartridge* (ID = 15 mm, resin bed height = 55 mm).

### Continuous flow esterification.

An HPLC pump was connected to the cartridge reactor 240 (volume of the cartridge containing the catalyst: 5 mL) from Ehrfeld Mikrotechnik BTS (cartridge filled with Amberlyst^{®} 15) via stainless steel tubing. A pressure sensor and a back-pressure regulator module were placed after the reactor. The outlet of the back pressure regulator was connected to a cartridge containing the scavenger. During optimization of the reaction, a sample injector (2 mL loop) was placed in the system.

### Experimental setup for optimization of reaction parameters

At first, a methanol/toluene mixture was pumped through the system and the system was pressurized to 15 bar. Then, the reactor temperature was set to the desired value. When the steady state was reached, a solution of 7-KCA in methanol/toluene mixture was circulated through the reactor. The exiting flow outstream was collected and an aliquot was analyzed by HPLC.

Optimization tests were started with the strong cation exchange resin DOWEX 50WX8. Effect of the temperature was examined with a 0.18 M substrate solution in a 2.5:1 (v/v) mixture of methanol / toluene, and with a flow rate of 1mL/min (Examples 1-3). As can be seen from Table 1, reaction conversion increased with temperature and reached 92.6% at 122°C.

**Table 1: Optimization of reaction parameters of temperature, solvent(s), concentration of the substrate, flow rate, catalyst for the esterification of 7-KCA with methanol under continuous flow conditions.**

| **Ex. No.** | **Ratio MeOH/ toluene (v/v)** | **[7-KCA] (mol/L)** | **Catalyst** | **Fluid temp. (°C)** | **Flow rate (mL/min)** | **Conversion (%)** |
|---|---|---|---|---|---|---|
| 1 | 2.5:1 | 0.18 | DOWEX 50WX8 | 94 | 1 | 61.3 |
| 2 | 2.5:1 | 0.18 | DOWEX 50WX8 | 108 | 1 | 81.2 |
| 3 | 2.5:1 | 0.18 | DOWEX 50WX8 | 122 | 1 | 92.6 |
| 4 | 3:1 | 0.16 | DOWEX 50WX8 | 122 | 1 | 94.5 |
| 5 | 3:1 | 0.16 | DOWEX 50WX8 | 122 | 0.8 | 97.4 |
| 6 | 3:1 | 0.16 | DOWEX 50WX8 | 122 | 0.7 | 97.6 |
| 7 | 3:1 | 0.16 | DOWEX 50WX8 | 122 | 0.5 | 97.5 |
| 8 | 4:1 | 0.13 | DOWEX 50WX8 | 122 | 0.8 | 96.1 |
| 9 | 4:1 | 0.10 | DOWEX 50WX8 | 122 | 0.8 | 97.8 |
| 10 | 4:1 | 0.06 | DOWEX 50WX8 | 122 | 0.8 | 98.1 |
| 11 | 3:1 | 0.16 | Amberlyst 15 | 122 | 0.8 | 98.2 |
| 12 | 3:1 | 0.16 | Amberlyst 15 | 113 | 0.8 | 98.1 |
| 13 | 3:1 | 0.16 | Amberlyst 15 | 132 | 0.8 | 97.5 |

With a slightly higher methanol/toluene ratio (3:1, v/v) and a lower substrate concentration (0.16 M), the conversion increased to 94.5% (Example 4). Further enhancement of the conversion was observed by reducing the flow rate, with a maximum attained at 0.7 mL/min (Examples 5-7). However, no significant differences were observed at 0.5, 0.7 and 0.8 mL/min.

At a constant methanol/toluene ratio (4:1, v/v), the reaction conversion increased from 96.1% to 98.1% by lowering substrate concentration from 0.13 M to 0.06 M (entries 8-10).

Another strong cation exchange resin, Amberlyst 15, was then evaluated. Experimental conditions of Example 5 were initially selected. Indeed, they represent a beneficial compromise between substrate concentration, flow rate and reaction conversion. Under these conditions, higher conversion was obtained with Amberlyst 15 compared to DOWEX 50WX8 (Example 11). Lowering the temperature from 122 to 113°C had almost no effect on the conversion (Example 12) but increasing to 132°C resulted in a decrease of the conversion (Example 13).

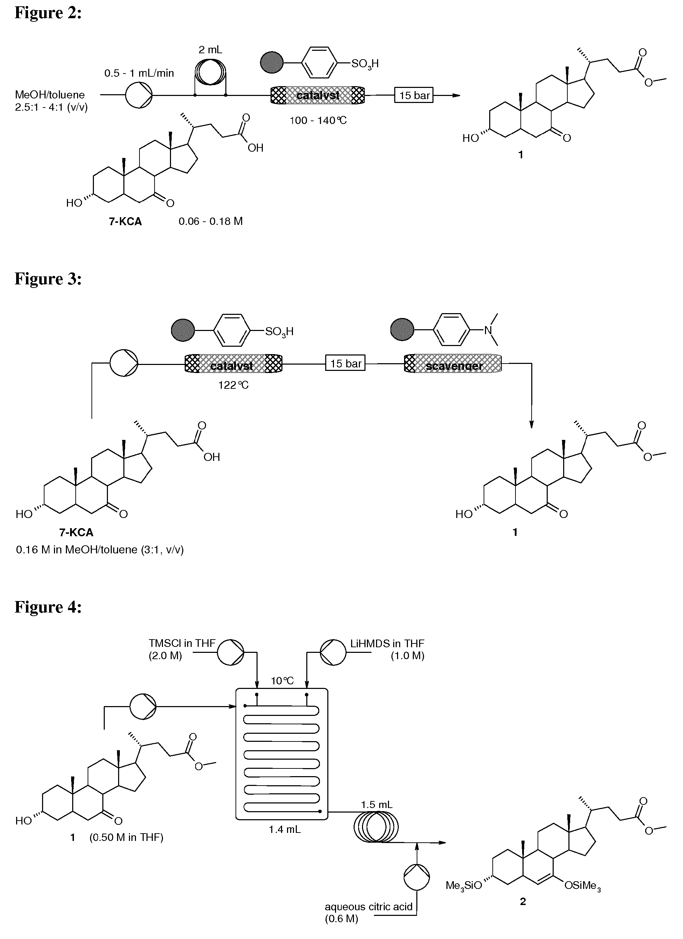

The above optimization of reaction parameters allowed to identify favorable conditions to perform the esterification of 7-KCA with methanol under continuous flow. However, full conversion couldn't be reached and 1.8-2.0% starting material were present in the final product. To overcome this finding, a process was developed using scavenging technique. Optimized experimental setup is depicted in Figure 3.

### Esterification of 7-KCA with methanol under optimized conditions

The equipment consists of an HPLC pump, the cartridge reactor 240 from Ehrfeld Mikrotechnik BTS (volume of the cartridge: 5 mL, filled with Amberlyst 15), a back pressure regulator and finally a cartridge (ID 15 mm, length 55 mm) containing the scavenger Amberlyst® A-21to remove traces of unreacted carboxylic acid 7-KCA from the process stream. The use of heterogeneous catalyst under flow conditions required only a minimal workup procedure. Indeed, the exiting flow outstream from the reaction could be concentrated directly to give the product in essentially quantitative yield with high chemical purity.

At first, a methanol/toluene mixture (3:1, v/v) was pumped through the system at 0.8 mL/min and the system was pressurized to 15 bar. Then, the reactor temperature was set to 130°C, resulting in a fluid temperature of about 122°C. When the steady state was reached, a solution of 7-KCA (42.3 mmol, 16.50 g) in 264 mL of methanol/toluene mixture (3:1, v/v) was circulated at 0.8 mL/min. The calculated residence time in the reactor cartridge was about 4.6 min. At the end, the system was flushed with 25 ml of methanol/toluene mixture (3:1, v/v). The exiting flow outstream was collected for 6 h and concentrated under reduced pressure. The residue was taken up with THF and concentrated again under reduced pressure giving 17.3 g of methyl ester 1 (quantitative yield, 98.9% purity).

### Step II: Preparation of 3α,7α-ditrimethylsilyloxy-5β-chol-6-en-24-oic acid methyl ester (2)

A continuous flow process for the synthesis of silyl ether / silyl enol ether of compound (1) was established, using a Modular MicroReaction System from Ehrfeld Mikrotechnik BTS.

Experimental setup consisted of a FlowPlate^{®} Lab reactor associated to a residence time unit (PTFE tubing, 1 mm I.D., 3 mm O.D.) and temperature-controlled by means of an external thermostat. Two HPLC pumps were used to circulate the substrate solution and the quenching mixture, whereas chlorotrimethylsilane and lithium hexamethyldisilazide solutions were introduced in the system via 2 syringe pumps made of chemically inert materials. The outlet of the residence time unit was connected to a T-shape connection module where quenching took place by pumping a 0.6 M citric acid aqueous solution at 0.83 ml/min. Optimized experimental setup is depicted in Figure 4.

Three feed solutions were connected to the FlowPlate^{®} Lab reactor:
- substrate solution (17.3 g in 85 ml dry THF, ~ 0.5 M) was circulated with an HPLC pump at 1 ml/min (inlet 1),
- chlorotrimethylsilane solution in dry THF (2.0 M) was introduced in the system via a syringe pump made of chemically inert materials at 1 ml/min (inlet 2),
- lithium hexamethyldisilazide solution in THF (1.0 M, commercially available) was also circulated in the system via a syringe pump at 2.5 ml/min (inlet 3).

At first, dry THF was pumped through the system at 1.0 mL/min and the FlowPlate^{®} Lab reactor temperature was set to 10°C by means of an external thermostat. When the steady state was reached, feed solutions and quenching solution were circulated through the system at the above mentioned flow rate. At the end, the system was flushed with 20 ml of dry THF. The exiting flow outstream was collected in a flask placed into an icebath. The mixture was concentrated under reduced pressure. The residue was taken up twice with 100 ml 2-MeTHF and concentrated again under reduced pressure giving 38.35 g of crude compound 2 which was used in the next step without further purification.
**Selected ¹H NMR** (400 MHz, CDCl₃) δ ppm: 0.12 (br s, 9 H) 0.69 (br s, 3 H) 0.83 (br s, 3 H) 0.93 (br s, 3 H) 2.17 - 2.30 (m, 1 H) 2.31 - 2.43 (m, 1 H) 3.46 - 3.57 (m, 1 H) 3.67 (s, 3 H) 4.74 (br s, 1 H).

**Table 2: Optimization of reaction parameters (temperature, stoichiometry, residence time) for the formation of compound (2) under continuous flow.**

| **Parameters** | **Value** |
|---|---|
| Flow rate of substrate solution (0.5 M in THF) | 1 ml/min |
| Flow rate of TMSC1 solution (2.0 M in THF) | 1 ml/min (4 eq.) |
| Flow rate of LiHMDS solution (1.0 M in THF) | 2.5 ml/min (5 eq.) |
| Flow rate of citric acid solution (0.6 M in water) | 0.83 ml/min (1 eq.) |
| Reactor temperature | 10°C |
| Residence time unit temperature | RT |

Under these conditions, a clean and complete conversion of compound (1) into compound (2) was observed (monitored by TLC).

### Step III: Preparation of 3α-hydroxy-6-ethyliden-7-oxo-5β-cholan-24-oic acid methyl ester (3)

A process for the continuous flow Mukaiyama aldol reaction of compound (2) with acetaldehyde was developed.

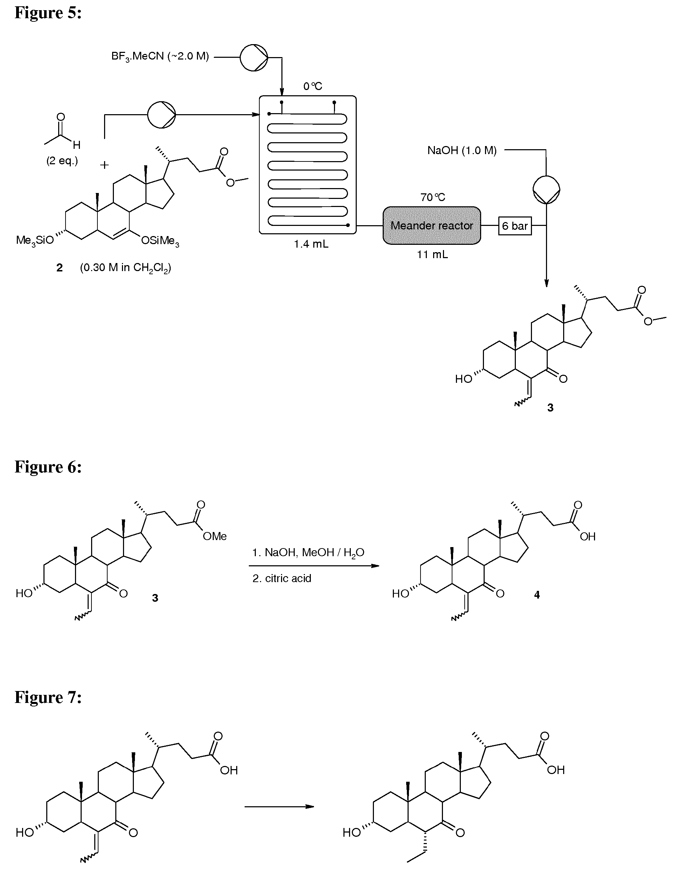

Experimental setup consisted of a Modular MicroReaction System from Ehrfeld Mikrotechnik BTS including a FlowPlate^{®} Lab reactor associated to a Meander (residence) reactor (11 ml process volume), both temperature-controlled by means of external thermostats. A pressure sensor and a back-pressure regulator module were placed after the reactor, to pressurize the system to 6 bar. Reactants solution and boron trifluoride solution were circulated with syringe pumps; the quenching solution was introduced in the system via an HPLC pump. The outlet of the back pressure regulator was connected to a T-shape connection module where quenching took place by pumping a 1 M sodium hydroxide at 0.91 ml/min. Optimized experimental setup is depicted in Figure 5.

Two feed solutions were connected to the FlowPlate^{®} Lab reactor:
- a solution containing the substrate (38.35 g of crude compound 2, ~ 0.3 M) and acetaldehyde (4.80 ml, 2 eq.) in 100 ml DCM was circulated with a syringe pump at 1 ml/min (inlet 1),
- 2 M boron trifluoride acetonitrile complex solution (commercially available) was introduced in the system via a syringe pump made of chemically inert materials at 0.46 ml/min (inlet 2).

At first, dichloromethane was pumped through the system at 1.0 mL/min and the system was pressurized to 6 bar. Then, the FlowPlate^{®} Lab reactor temperature and the residence reactor temperature were set to 0°C and 70°C, respectively. When the steady state was reached, both feed solutions and quenching solution were circulated through the system at the above mentioned flow rate. At the end, the system was flushed with 20 ml of DCM. The exiting flow outstream was collected in a flask at room temperature. After decantation, the organic phase was washed with brine (2 x 150 ml) and concentrated under reduced pressure giving 16.45 g of crude compound 3 which was used in the next step without further purification.
**Selected ¹H NMR** (400 MHz, CDCl₃) δ ppm: 0.69 (s, 3 H) 0.96 (d, *J* = 6.4 Hz, 3 H) 1.04 (s, 3 H) 1.71 (d, *J* = 7.1 Hz, 3 H) 2.64 - 2.73 (m, 1 H) 3.54 - 3.63 (m, 1 H) 3.65 (s, 3 H) 4.58 (br s, 1 H) 6.08 (q, *J* = 7.1 Hz 1 H).

**Table 3: Optimization of reaction parameters (temperature, stoichiometry, residence time) for the formation of compound (3) under continuous flow.**

| **Parameters** | **Value** |
|---|---|
| Flow rate of reactants solution (0.3 M of 2 / 0.6 M acetaldehyde in CF₂Cl₂) | 1 ml/min |
| Flow rate of BF₃.MeCN solution (2.0 M BF₃ in MeCN) | 0.46 ml/min (3 eq.) |
| Flow rate of NaOH solution (1.0 M in water) | 0.91 ml/min (3 eq.) |
| FlowPlate® Lab reactor temperature | 0°C |
| Meander reactor temperature | 70°C |
| Back-pressure regulator | 6 bar |

Under these conditions, compound 3 was predominantly formed with a purity of about 85% (determined by HPLC analysis, at 200 nm, sum of both isomers). This crude material was directly engaged in the next step without further purification.

### Step IV: Preparation of 3α-hydroxy-6-ethyliden-7-oxo-5β-cholan-24-oic acid (4)

Saponification of methyl ester (3) with sodium hydroxide, in a water / methanol mixture, followed by acidification with citric acid led to compound 4 in 50% yield from 7-KCA (4-steps) with a chemical purity of 93% (sum of E and Z isomers).

A 250 ml flask was charged with crude compound (3) (16.40 g), methanol (30 ml) and water (3.5 ml). A 50% sodium hydroxide solution (3.5 ml) was added dropwise at RT (25°C), then the mixture was heated to 50°C and stirred until completion (about 2 hours). The mixture was allowed to cool down to RT, diluted with water (70 ml) and decanted. The aqueous phase was washed with ethyl acetate (1 x 50 ml) then transferred into a 500 ml flask. A solution of citric acid (15.51 g) in water (30 ml) was added dropwise followed by ethyl acetate (90 ml). After 5 min of vigorous stirring, the mixture was decanted and the organic phase concentrated under reduced pressure. The residue was taken up with ethyl acetate (50 ml) and concentrated as before. The yellow/orange solid was stirred in acetone (20 ml) at RT for 30 min then cooled to 10°C. The precipitate was collected by filtration, washed with cold acetone (2 x 5 ml) and dried at 50°C / 10 mbar to give 7.87 g of compound 4 as yellow solid (1^{st} fraction).

The mother liquor was concentrated under reduced pressure. The yellow/orange solid was taken up with ethyl acetate (15 ml) and stirred at RT for 30 min then cooled to 10°C. The precipitate was collected by filtration, washed with ethyl acetate (2 x 5 ml) and dried at 50°C / 10 mbar to give 0.96 g of compound (4) as yellow solid (2^{nd} fraction). Both isolated fractions of compound (4) were mixed together leading to the following results: 50% overall yield (over 4 steps) and 93% purity (sum of E and Z isomers).
HPLC (OCA01.M): 3.05 min (88.3%, major isomer), 3.26 min (4.6%, minor isomer) (200 nm).
LC-MS (OBETICHOLIC ACID-PH2-POLAR-100-5.M): 4.97 min (m/z = 415, [M-H]⁻, major isomer), 5.20 min (m/z = 415, [M-H]⁻, minor isomer).
Selected ¹H NMR (400 MHz, CDCl₃) δ ppm: 0.65 (s, 3 H), 0.94 (d, *J* = 6.4 Hz, 3 H), 1.01 (s, 3 H), 1.69 (d, *J* = 6.4 Hz, 3 H), 2.3 - 2.5 (m, 2 H), 2.58 (dd, *J* = 13.0, 4.2 Hz, 1 H), 3.6 - 3.7 (m, 1 H), 6.18 (q, *J* = 7.0 Hz, 1 H).
¹³C NMR (400 MHz, CDCl₃) δ ppm: 12.10, 12.72, 18.44, 21.36, 22.86, 25.98, 28.43, 29.69, 30.82, 31.06, 34.49, 34.61, 35.24, 37.48, 38.99, 39.13, 43.63, 45.55, 48.73, 50.70, 54.57, 70.57, 129.94, 143.33, 179.56, 204.95.

### Step V: Preparation of 3α-hydroxy-6α-ethyl-7-keto-5β-cholanic acid (obeticholic acid)

Exocyclic double bond hydrogenation with H₂ and Pd/C was carried out in an alkaline reaction medium under heating in order to allow the epimerization of the 6β-ethyl group into 6α-ethyl, as mentioned in WO 2006/122977 (p. 10). It afforded 3α-hydroxy-6α-ethyl-7-keto-5β-cholan-24-oic acid in about 83% yield.

An autoclave was charged with 2.00 g of 3α-hydroxy-6-ethyliden-7-keto-5β-cholanic acid (4.80 mmol, 1.00 eq.) and 20 ml water. Then, 0.65 ml of 50% sodium hydroxide (12.4 mmol, 2.59 eq.) was added dropwise at RT followed by 0.20 g of 10 Pd/C (0.05 mmol, 0.01 eq.). The system was purged with N₂ (3 x 2 bar) then with H₂ (3 x 5 bar). The system was pressurized with 5 bar H₂ and the temperature was raised to 105°C (oil-bath temperature) over 90 min. After stirring at 105°C for 3 h, the system was allowed to cool down to 40°C within 1 h and the gas was carefully released. The mixture was filtered over glas-fiber filter and the filtrate was diluted with 24 ml n-butyl acetate. Concentrated HCl (37%, 1.24 ml, 14.8 mmol, 3.09 eq.) was added dropwise at RT. After 5 min vigorous stirring, the mixture was decanted. Activated charcoal (0.1 g) was added to the organic phase and the mixture was stirred at 40°C for 10 min. After filtration, the mixture was concentrated under reduced pressure. The white solid was taken up with 8 ml n-butyl acetate. The slurry was cooled to 10-15°C and filtered. The solid was washed with cold n-butyl acetate (2 x 3 ml) and dried at 50°C/10 mbar for 20 h, yielding 1.66 g of 3α-hydroxy-6α-ethyl-7-keto-5β-cholanic acid as off-white solid (82.6%).
**Selected ¹H NMR** (400 MHz, MeOD) δ ppm: 0.71 (s, 3 H) 0.82 (d, *J* = 7.4 Hz, 3 H) 0.96 (d, *J* = 6.7 Hz, 3 H) 1.26 (s, 3 H) 2.28 - 2.39 (m, 1 H) 2.50 (t, *J* = 11.3 Hz, 1 H) 2.83 (dt, *J* = 8.2, 5.5 Hz, 1 H) 3.41 - 3.51 (m, 1 H).

### Step VI: Preparation of crystalline obeticholic acid, Form C

Sodium borohydride reduction of the carbonyl group followed by acidification with citric acid led to obeticholic acid in about 81% yield, which crystallized from *n*-butyl acetate.

A 250 ml RBF was charged with 9.00 g of 3α-hydroxy-6α-ethyl-7-keto-5β-cholanic acid (21.5 mmol, 1.00 eq.) and 72 ml water. Then, 5.91 ml of 50% sodium hydroxide (0.113 mol, 5.26 eq.) was added dropwise at RT. In parallel, 0.81 g NaBH₄ (21.5 mmol, 1.00 eq.) was dissolved in a mixture of water (2.13 ml) and 0.16 ml of 50% NaOH (3.00 mmol, 0.14 eq.) then added dropwise at RT. The resulting mixture was stirred at reflux and the progression of the reaction was followed by TLC. After 2 h, another portion of NaBH₄ (0.41 g) solution in water (1.1 ml) and 50% NaOH (0.1 ml) was added and the mixture further stirred at reflux. After 2 h, the mixture was cooled down to about 40°C and diluted with 90 ml n-butyl acetate. A solution of citric acid (33.6 g in 52 ml water) was added dropwise and the resulting mixture was vigorously stirred for 10 min. After decantation, the organic phase was concentrated under reduced pressure. The oily residue was taken up with 60 ml n-butyl acetate. The slurry was cooled to 15-20°C with stirring then filtered. The solid was washed with cold n-butyl acetate (4 x 5 ml) and dried at 50°C/10 mbar overnight, yielding 7.30 g of obeticholic acid as white solid (80.7%).
**HPLC/MS** (OBETICHOLIC ACID-PH7-POLAR-1.M): 3.8 min (m/z = 839.6 [2M-H]⁻, 479.2 [M+CH₃CO₂H-H]⁻, 419.3 [M-H]⁻).
**Selected ¹H NMR** (400 MHz, MeOD) δ ppm: 0.69 (s, 3 H) 2.14 - 2.26 (m, 1 H) 2.27 - 2.39 (m, 1 H) 3.63 - 3.69 (m, 1 H) 4.05 (t, *J* = 6.7 Hz, residual n-butyl acetate, ~ 67000 ppm).
**XRPD:** crystalline, some similarities with known Form C from WO 2013/192097.

### Step VII: Preparation of amorphous obeticholic acid, Form 1.

Conversion of crystalline obeticholic acid Form C to amorphous obeticholic acid Form 1 was done by converting it into a sodium salt followed by neutralization with hydrochloric acid. Yield of this transformation was about 91% and XRPD analysis confirmed the formation of amorphous product. Chemical purity of obeticholic acid was very high, almost 100% according to HPLC/UV analysis.

A 25 ml RBF was charged with 7.00 g of obeticholic acid Form C (16.6 mmol, 1.00 eq.) and 105 ml water. Then, 1.05 ml of 50% sodium hydroxide (20.1 mol, 1.21 eq.) was added dropwise at 30 - 40°C and the mixture was stirred until all solids have dissolved. Afterwards, it was added slowly to a solution of 37% hydrochloric acid (2.00 ml, 23.4 mmol, 1.41 eq.) in water (105 ml), at 30 - 40°C. The resulting white slurry was stirred for 30 min at 30 - 40°C then cooled to 20°C and filtered. The solid was washed with water (6 x 20 ml) and dried at 50°C/7 mbar overnight, yielding 6.40 g of obeticholic acid Form 1 as white solid (91.4%).
**HPLC** (OCA01.M): 4.57 min (100%) (200 nm).
**LC/MS** (OBETICHOLIC ACID-PH7-POLAR-1.M): 3.8 min (m/z = 839.7 [2M-H]⁻, 479.3 [M+CH₃CO₂H-H]⁻ 419.3 [M-H]⁻).
**Selected ¹H NMR** (400 MHz, MeOD) δ ppm: 0.70 (s, 3 H) 0.87 - 0.94 (m, 6 H) 0.97 (d, *J*=6.26 Hz, 3 H) 1.98 - 2.04 (m, 1 H) 2.15 - 2.25 (m, 1 H) 2.28 - 2.39 (m, 1 H) 3.64 - 3.68 (m, 1 H).
**¹³C NMR** (400 MHz, CDCl₃) δ ppm: 12.18, 12.38, 18.95, 22.13, 23.66, 23.91, 24.72, 29.43, 31.41, 32.14, 32.50, 34.59, 34.68, 36.80, 36.92, 41.20, 41.71, 43.30, 43.89, 47.12, 51.82, 57.52, 71.33, 73.35, 178.32.
**Selected IR peaks** (cm⁻¹): 3437, 2933, 2870, 1707, 1450, 1377, 1063.
**TGA:** 0.6% weight loss between 25°C and 75°C.
**Residual solvent** (Headspace): n-butyl acetate (192 ppm).

### Advantages of the invention

Hence, it has surprisingly been found that the reaction setup as used in the prior art batch processes can be much simplified and process efficiency can be increased by performing one or more process steps in a continuous flow reactor, i.e. as continuous flow process steps.

In particular, performing one or more process steps in the process for preparing obeticholic acid in a continuous flow reactor has the advantage that the reaction temperature can be increased, even to temperatures above the boiling point at 1013 hPa of the solvents used in the respective process step. Thus, the reaction efficiency can be increased, leading to shortened reaction times and increased product yield.

In summary, it could surprisingly be found in the present invention that using the continuous flow technology for the preparation of obeticholic acid has advantages of simplification of the production processes, such as improved process monitoring and reproducibility, which may also lead to a significant reduction of production costs. Further benefits are increased yields of obeticholic acid, high product quality, a significant reduction in waste, a decrease in the demand of raw materials, as well as needing much less energy for downstream processing of reaction mixtures.

## Claims

1. A process for the preparation of 3α,7β-dihydroxy-6a-ethyl-5β-cholan-24-oic acid (obeticholic acid) or one of its process intermediates selected from 3α-hydroxy-7-oxo-5β-cholan-24-oic acid C₁₋₄alkyl ester (1), 3α,7α-ditrimethylsilyloxy-5β-chol-6-en-24-oic acid C₁₋₄alkyl ester (2), 3α-hydroxy-6-ethyliden-7-oxo-5β-cholan-24-oic acid C₁-₄alkyl ester (3) and 3α-hydroxy-6-ethyliden-7-oxo-5β-cholan-24-oic acid (4), the process starting from 3α-hydroxy-7-keto-5β-cholanic acid (7-KCA) as starting material and comprising one or more continuous flow process steps as represented by formulae (1) to (4).

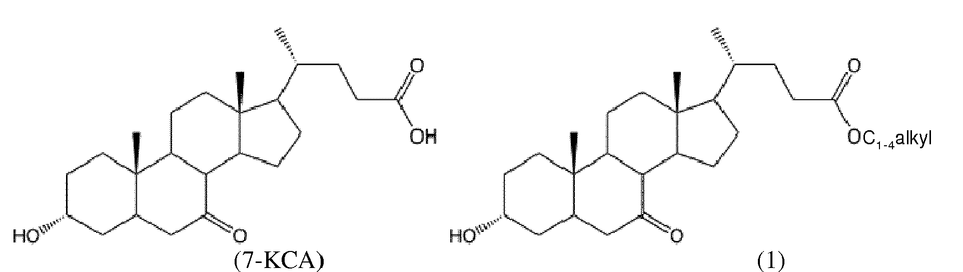

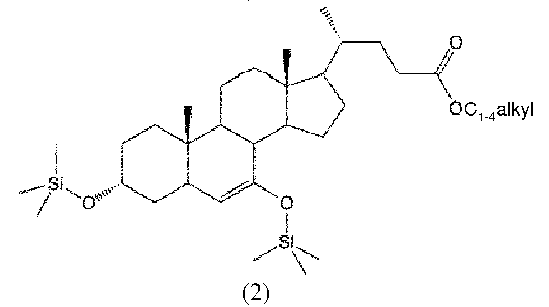

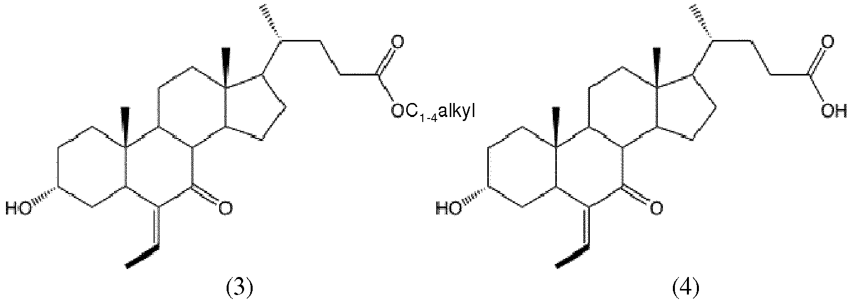

2. The process of claim 1, comprising the following process steps:
(I) esterification of 3α-hydroxy-7-keto-5β-cholanic acid (7-KCA) with a C₁₋₄alkyl alcohol to form 3α-hydroxy-7-oxo-5β-cholan-24-oic acid C₁₋₄alkyl ester (1),
(II) reacting (1) with a silylation agent and a lithium amide base to form 3α,7α-ditrimethylsilyloxy-5β-chol-6-en-24-oic acid C₁₋₄alkyl ester (2),
(III) reacting (2) with acetaldehyde or acetaldehyde acetal and a Lewis acid catalyst to form 3α-hydroxy-6-ethyliden-7-oxo-5β-cholan-24-oic acid C₁₋₄alkyl ester (3),
(IV) reacting (3) with sodium hydroxide to form 3α-hydroxy-6-ethyliden-7-oxo-5β-cholan-24-oic acid (4), and
(V) optionally converting (4) into obeticholic acid.

3. The process of claim 2, wherein steps (I) to (III) are performed as continuous flow process steps.

4. The process of claim 2 or 3, wherein esterification in step (I) is conducted in a flow device, comprising at least a feed solution inlet, a heatable cartridge comprising a heterogeneous catalyst and a backpressure regulator,
wherein 3α-hydroxy-7-keto-5β-cholanic acid (7-KCA) in a mixture of alcohol and an organic solvent is fed to the flow reactor inlet.

5. The process of claim 4, wherein the heterogeneous catalyst is a cation exchange resin containing sulfonic acid functional groups.

6. The process of any one of claims 2 to 5, wherein the alcohol is methanol and the organic solvent is toluene and the weight ratio of methanol:toluene is from 2.5:1 to 4:1.

7. The process of any one of claims 2 to 6, wherein the reaction mixture leaving the flow reactor of step (I) is contacted with a basic anion exchanger containing tertiary C₁₋₁₀-alkyl amine functional groups.

8. The process of any one of claims 2 to 7, wherein step (I) is performed at a temperature of from 100°C to 140°C under a pressure of from 10 bar to 15 bar.

9. The process of any one of claims 2 to 8, wherein silylation in step (II) is conducted in a flow device, comprising at least a first reactor unit with at least three feed solution inlets, a second reactor unit and a 3-way connection module, the process comprising:
feeding 3α-hydroxy-7-oxo-5β-cholan-24-oic acid C₁₋₄alkyl ester (1) in an organic solvent to a first flow reactor inlet;
feeding a silylation agent in an organic solvent to a second flow reactor inlet;
feeding lithium amide base in an organic solvent to a third flow reactor inlet; and
feeding an acidic aqueous solution to the 3-way connection module.

10. The process of any one of claims 2 to 9, wherein the silylation agent in step (II) comprises chlorotrimethylsilane, the lithium amide base is selected from lithium hexamethyldisilazide, lithium diisopropylamide, lithium 2,2,6,6-tetramethylpiperidide, and mixtures thereof, and/or the organic solvent is selected from tetrahydrofuran, 2-methyl tetrahydrofuran, toluene, xylene, anisole and mixtures thereof.

11. The process of any one of claims 2 to 10, wherein step (II) is performed at a temperature of from -20°C to 40°C.

12. The process of any one of claims 2 to 11, wherein the reaction in step (III) is conducted in a flow device, comprising at least a first reactor unit with at least two feed solution inlets, a residence reactor unit and a 3-way connection module, the process comprising:
feeding 3α,7α-ditrimethylsilyloxy-5β-chol-6-en-24-oic acid C₁₋₄alkyl ester (2) and acetaldehyde or acetaldehyde acetal in an organic solvent to a first flow reactor inlet;
feeding the Lewis acid catalyst in an organic solvent to a second flow reactor inlet; and
feeding sodium hydroxide aqueous solution to the 3-way connection module.

13. The process of claim 12, wherein a reaction temperature in the first reactor unit is from -20°C to 20°C, and a reaction temperature in the residence reactor unit is from 50°C to 90°C.

14. The process of any one of claims 2 to 13, wherein the acetaldehyde acetal in step (III) is selected from acetaldehyde dimethyl acetal or acetaldehyde diethyl acetal, the Lewis acid catalyst is selected from boron trifluoride complex with an organic solvent or titanium tetrachloride, and/or the organic solvent is selected from dichloromethane, acetonitrile, tetrahydrofuran, 2-methyl tetrahydrofuran, toluene, xylene, anisole and mixtures thereof.

15. The process of any one of claims 12 to 14, wherein the reaction temperature of step (III) in the second reactor unit is above the boiling point of the solvent at 1013 hPa, and the pressure is set to maintain the solvent under its boiling point.

16. Use of a continuous flow reactor for the preparation of 3α,7β-dihydroxy-6α-ethyl-5β-cholan-24-oic acid (obeticholic acid) or one of its process intermediates selected from 3a-hydroxy-7-oxo-5β-cholan-24-oic acid C₁₋₄alkyl ester (1), 3α,7α-ditrimethylsilyloxy-5β-chol-6-en-24-oic acid C₁₋₄alkyl ester (2), 3α-hydroxy-6-ethyliden-7-oxo-5β-cholan-24-oic acid C₁₋₄alkyl ester (3) and 3α-hydroxy-6-ethyliden-7-oxo-5β-cholan-24-oic acid (4).
